Europäisches Patentamt

(19) European Patent Office

Office européen des brevets

(11) Publication number : **0 292 517 B1**

(12) **EUROPEAN PATENT SPECIFICATION**

(45) Date of publication of patent specification :
09.09.92 Bulletin 92/37

(21) Application number : 87907612.3

(22) Date of filing : 24.11.87

(86) International application number :
PCT/GB87/00839

(87) International publication number :
WO 88/04180 16.06.88 Gazette 88/13

(51) Int. Cl.⁵ : **A61K 37/36, A23K 1/18,
A23K 1/165, A23K 1/175,
// (A61K37/36, 33:26)**

(54) IMPROVEMENTS IN OR RELATING TO THE USE OF EPIDERMAL GROWTH FACTOR.

(30) Priority : 11.12.86 GB 8629674
05.06.87 GB 8713169

(43) Date of publication of application :
30.11.88 Bulletin 88/48

(45) Publication of the grant of the patent :
09.09.92 Bulletin 92/37

(84) Designated Contracting States :
AT BE CH DE FR IT LI LU NL SE

(73) Proprietor : BRITISH TECHNOLOGY GROUP
LTD
101 Newington Causeway
London SE1 6BU (GB)

(72) Inventor : WILSON, Thomas, James, Greer
22 Aylestone Road
Cambridge CB4 1HF (GB)

Inventor : TIVEY, David, Robert Cambridge
Lodge
Babraham
Cambridge CB2 4AF (GB)
Inventor : SMITH, Michael, Waddington
94 Glebe Road
Cambridge CB1 4TA (GB)
Inventor : JAMES, Peter, Stephen
60 Radegund Road
Cambridge CB1 3RS (GB)
Inventor : PETERS, Timothy, John
12 Cleveland Road
London W13 8AU (GB)
Inventor : RAJA, Kishor, Barchand 288
Headstone Lane
North Harrow
Middlesex HA2 6NE (GB)

(74) Representative : Stephenson, Gerald
Frederick et al
Patents Department British Technology Ltd
101 Newington Causeway
London SE1 6BU (GB)

EP 0 292 517 B1

# Description

This invention relates to animal husbandry and to human and veterinary medicine, particularly to compositions such as pharmaceutical compositions and foodstuffs containing iron and their use.

An adequate supply of iron to the body is an essential requirement for tissue growth in both man and animals. Although there is normally an ample amount of iron in the diet, the level of absorption of iron from food is generally low so that the supply of iron to the body can easily become critical under a variety of conditions. Iron deficiency anaemia is commonly encountered in pregnancy and may also present a problem in neonates or the newly born, particularly in certain animal species such as the pig. Moreover, in certain pathological conditions there is a malabsorption or maldistribution of body iron leading to a state of chronic anaemia. Such malabsorption or maldistribution is seen in certain intestinal disorders and in chronic diseases such as rheumatoid arthritis, certain haemolytic diseases and cancer.

Although a wide range of iron compounds is marketed for the treatment of iron deficiencies and the results thereof, and for the prophylaxis of such iron-deficiency states, the level of iron uptake by the body from these compounds is often quite low thereby necessitating the administration of relatively high dosage levels of the compound. The administration of high dose, poorly absorbed, iron complexes may cause siderosis of the gut wall and a variety of side effects such as nausea, vomiting, constipation and heavy malodorous stools.

It is therefore an object of the present invention to provide a means of enhancing the uptake of iron and it has been found that this may be achieved through the use of epidermal growth factor (EGF), usually through the incorporation into a pharmaceutical composition or a foodstuff of EGF. Although therapeutic applications of EGF have previously been described, for example in UK Patent 1,417,776 which describes the use of EGF in inhibition of the secretion of acidic gastric juice, there has never previously been any indication that EGF had a role in the enhancement of iron uptake by the body.

Accordingly the present invention comprises a composition suitable for use in vivo to enhance the level of iron in the bloodstream comprising epidermal growth factor and an iron-providing material.

The present invention extends to the use of EGF in pharmaceutical compositions and foodstuffs as described hereinafter to enhance iron uptake from a wide variety of iron-providing materials, which may contain iron in the ferrous or particularly the ferric form, in both humans, animals and birds. Particular interest centres on the treatment of mammals, especially humans and also pigs, for example piglets.

The iron-providing material may be any physiologically acceptable substance capable of raising the level of iron in the bloodstream on administration in vivo, including both iron salts and iron complexes. Examples of specific iron-providing materials include, particularly for human use, ferric chloride, ferric ascorbate, ferric citrate, ferrous fumarate, ferrous gluconate and ferrous succinate, and, particularly for use in piglets, compounds, including some of those mentioned above, which are described in UK Patent 1,322,102 and US Patent 4,362,710, for example iron (ferric) dextran, ferrous fumarate and ferric citrate. Also of particular interest for both human and animal use are the iron complexes which are the subject of UK Patents and Patent Applications 2117766B, 2136806A, 2157686A and particularly 2128998B, especially (3-hydroxy-2-methyl-4-pyrone)$_3$ iron(III) and related homogeneous and heterogeneous 3:1 hydroxypyrone:iron(III) complexes.

The EGF may be administered alone in order to enhance iron uptake from either normally ingested or specifically administered iron-providing materials and in the latter aspect the present invention therefore includes a product comprising epidermal growth factor and an iron-providing material for simultaneous, separate or sequential use in effecting an increase in iron levels in a patient's bloodstream, for example a product comprising the two components in association. However, it will most usually be formulated together with an iron-providing material and the present invention therefore includes a product comprising an iron-providing material and epidermal growth factor for use in therapy.

The EGF used in the present invention may conveniently be derived from various natural sources, particularly mammalian sources, since the active sequence thereof is closely conserved among species. Alternatively, synthetic EGF may be used that may optionally have variations in the molecule, within the active sequence or particularly outside it, which do not correspond to those found in nature or which constitute an admixture of variations found in different species in nature. Essentially any compound expressing the activity of the natural hormone may be used. Accordingly, the term epidermal growth factor is therefore used herein in a general sense to include all such natural materials and their synthetic equivalents, as well as variants thereon retaining the physiological activity of the molecule. Specific sources include mouse, rat, rabbit, cattle, goat, sheep, horse, pig and human EGF (urogastrone). In principle, EGF of the same species as the recipient is to be preferred and pig and human EGF are therefore of particular interest. Also of some especial interest is mouse EGF which has been produced by genetic engineering, although this technique could also be applied to the production of pig and human EGF, etc, the terms pig EGF and mouse EGF, for example, being used in a general sense to include the natural material and its synthetic equivalent, as well as variants thereon retaining the physiological activity of the molecule. The structure of mouse EGF is shown in UK Patent 1,417,776 and examples of possible active variants of the natural structure are also shown therein illustrating the type of variation which may also be applied with EGF derived from other sources whilst retaining activity.

EGF may be used according to the present invention for the manufacture of medicaments having a variety of forms. Usually, however, these will comprise, in addition to the EGF and an iron-providing material, a physiologically acceptable diluent or carrier. The present invention therefore includes a pharmaceutical composition comprising an iron-providing arterial, epidermal growth factor and a physiologically acceptable diluent or carrier.

The iron-providing material and the EGF may be formulated together in a pharmaceutical composition by a variety of methods. For instance, they may be applied as an aqueous, oily or emulsified composition incorporating a liquid diluent which may often be employed in injectable form for parenteral administration and therefore may conveniently be sterile and pyrogen free. For certain other uses a diluent which is sterile but not necessarily pyrogen free may be appropriate. As regards liquid diluents or carriers therefore, there is often particular interest in those which are sterile. Oral administration is often preferred for the treatment of iron deficiency anaemia in humans and the present invention is suited to such a route of administration. Although compositions incorporating a liquid diluent may be used for oral administration, it is more usual, at least in humans, to use compositions incorporating a solid carrier, for example a conventional solid carrier material such as starch, lactose, dextrin or magnesium stearate. Such solid compositions may conveniently be of a formed type, for example as tablets, capsules (including spansules), etc.

As indicated, liquid compositions are of particular interest in relation to parenteral administration, a requirement for which arises with humans in certain contexts but also particularly in a veterinary context, for example with pigs. The problems of iron deficiency anaemia in newly born pigs arise primarily during the first three weeks or so of their life when a very rapid weight gain takes place. The usual routes for administration of iron-providing materials to young piglets in the context of the present invention are parenteral, for example intramuscular, or oral, for example as a liquid preparation "injected" into the mouth. However, an alternative approach is to enhance the iron content of the milk on which the piglets are feeding by treating the mother pig using oral or parenteral administration, for example with an injectable slow release preparation (such an approach may also be of interest in a human context). Since EGF has only a short half life in vivo when given parenterally (about 2½ minutes when given intravenously) it is appropriate either to administer a series of doses during the period of treatment, for example at eight hour intervals or, preferably, to administer the EGF in the form of a controlled release preparation (including a pulsed release preparation), for example in a liposome preparation such as is described in EP-A-0007714 or in a polymeric preparation such as is described in UK Patent 2112381, from which the EGF is released during the required time period. Other alternatives for effecting a gradual release of the EGF include implants (polymers and waxes), mineral oil formulations, colloidal suspensions and the use of forms of EGF of low solubility.

Other forms of administration than by injection or through the oral route may also be considered in both human and veterinary contexts, for example the use of suppositories or pessaries, or of compositions for buccal or nasal administration. Further details regarding the formulation of iron-providing materials are to be found in the patents relating to such compounds mentioned hereinbefore.

The compositions may be formulated in unit dosage form, i.e. in the form of discrete portions containing a unit dose, or a multiple or sub-unit of a unit dose. The dosage of the iron-providing material will of course depend on the particular material which is used but it may be indicated by way of guidance that the daily requirement of iron for the adult human is generally regarded as being from 2 to 4 mg and that the dosage is therefore that which is appropriate to ensure this level of intake. Further information on the dosage levels of the iron compounds is to be found in the ABPI Data Sheet Compendium published annually by Datapharm Publications Ltd., london, U.K., and in the various patents and patent applications mentioned hereinbefore. As regards the EGF, dosage will again depend on the particular iron-providing material to be used in conjunction with the EGF but, as a guide, it may be stated that an appropriate range is from 1.0 to 100 in terms of n.moles EGF/n.mole iron present in the material. The appropriate ratio will of course depend on the efficiency of the material as an iron provider in vivo and ratios both below and above those quoted may be considered. By way of further guidance it may be stated that in the context of the present invention a daily dosage of EGF of 10 to 100 n.moles/kg of body weight is often appropriate, although once again doses outside this range may be considered.

In addition to its use in pharmaceutical compositions the incorporation of EGF into foodstuffs may be considered, usually those already containing a source of iron. Such foodstuffs may take various forms, either liquid, semi-solid or solid, and may for example take the form of conventional human infant or piglet feed materials. Further examples of such piglet feed materials are to be found in the US Patent 4,362,710 referred to hereinbefore.

The present invention thus further includes a foodstuff which comprises epidermal growth factor and an iron-containing nutritional material.

Moreover, it will also be appreciated that the present invention includes the use of epidermal growth factor for the manufacture of a medicament for use in effecting an increase in the level of iron in a patient's bloodstream.

The use of EGF is also of interest in conjunction with other materials than iron-providing materials which are of value specifically in growth promotion or in maintaining general well being of a human or an animal, particularly a piglet. One group of such materials is those which provide one of the fifteen trace elements essential for human and animal well being. Many of these are transition elements and apart from iron other metallic trace elements of particular interest are chromium, and also manganese, cobalt, copper and molybdenum, as well as selenium and zinc. Pharmaceutical compositions and foodstuffs according to the present invention may therefore, in addition to EGF and an iron-providing material, contain a material providing one of these . other trace elements or a plurality of materials providing different elements, particularly of one or more of cobalt, copper, selenium and zinc. Such materials may conveniently be chosen from those described in the art for providing the element in question and the pharmaceutical composition or foodstuff may conveniently, particularly in the case of a foodstuff, contain materials providing iron and a range of these beneficial trace elements. Data on preferred human and veterinary dosage levels for the different elements is to be found in the literature but the use of daily dosages of EGF selected within the ranges quoted herein together with proportions of EGF and the element in question within the range quoted herein for iron will usually be broadly suitable.

The invention is illustrated by the following Example.

## EXAMPLE

### Enhancement of Uptake of Iron in Mice

(A) Six week old, male To mice received doses averaging 1 n.mole/day of mouse epidermal growth factor in their drinking water during a period of up to 7 days, a control group of similar mice receiving plain drinking water. Iron absorption was then studied in the mice by both in vitro and in vivo techniques. The former, using techniques as described by Raja et al., Cell Biochem. and Funct., 1987, 5, 69-76, involved incubation of intestinal fragments from the mice with a 0-450 $\mu$M solution of a $Fe^{3+}$ chelate (the 2:1 nitrilotriacetic acid:iron(III) complex) whilst the latter, using techniques as described by Simpson and Peters, Biochim. Biophys. Acta., 1986, 856, 115-122, involved instillation of 50-100 $\mu$l of a 250 $\mu$M solution of the $Fe^{3+}$ chelate into a tied-off loop of intestine of an anaesthetised mouse.

It was found that neither group showed a change in the wet weight of the duodenum per unit length but that cell turnover rates, as reflected by L-ornithine decarboxylase activity, were elevated in the duodenum of the animals which had received EGF. In vitro uptake studies also showed no change in the kinetic parameters for $^{59}Fe^{3+}$ uptake for EGF-treated animals [$K_m$ = 101 $\pm$ 18(4); $V_{max}$ = 9.4 $\pm$ 1.1(4) pmol/mg/min] as compared with the controls [$K_m$ = 103 $\pm$ 20(9); $V_{max}$ = 10.5 $\pm$ 0.9(9)]. In vivo experiments showed a progressive increase in the total mucosal uptake of $^{59}Fe^{3+}$ in the EGF-treated animals which was maximal after 3 days of EGF administration. The data obtained is shown in the Table from which it will be seen that the enhanced uptake was due to increases in both the mucosal retention and carcass transfer.

In vivo studies were carried out with $^{51}$Cr-EDTA (ethylene diamine tetra-acetic acid) using techniques as described by Bjamason et al., Gut, 1985, 26, 579-586 which involve instillation of 50-100 $\mu$l of a 100 $\mu$M $^{51}$Cr-EDTA solution into a tied-off loop of intestine of an anaesthetised mouse. These showed an increased permeability in EGF-treated animals [total mucosal uptake = 25.1 $\pm$ 1.7(5) pmol/ mg/10 min] as compared with the controls (total mucosal uptake = 9.6 $\pm$ 1.1(5) pmol/mg/10 min; p < 0.001].

These studies demonstrate that oral EGF feeding enhances intestinal proliferation and in vivo $^{59}Fe^{3+}$ absorption, although the latter is not via a specific carrier-mediated pathway.

TABLE[1]

| | Mucosal retention | Carcass transfer | Total mucosal flux |
|---|---|---|---|
| Controls(20) | $40.6 \pm 2.3$ | $22.9 \pm 2.0$ | $63.4 \pm 3.4$ |
| EGF-treated | $57.1 \pm 6.3$[2] | $35.3 \pm 2.6$[2] | $92.4 \pm 8.4$[3] |

(1) Values are for mean $\pm$ SE pmol $^{59}Fe^{3+}$/mg tissue/10 minutes for number of animals indicated between parentheses.

(2) $p < 0.01$

(3) $p < 0.001$

(B) The procedure described under (A) was repeated but with the treated mice receiving the EGF for 3 rather than 7 days. After fasting for 12 hours both the EGF-treated and the control mice were treated intragastrically with 1 µCurie of $^{59}Fe^{3+}$ administered as 50 µl of a 100 µM aqueous solution of the radiolabelled 2:1 nitrilotriacetic acid:iron(III) complex. The mice were subjected to a whole body count by gamma counting at 3 hours and 7 days later. The percentage of the $^{59}Fe^{3+}$ present at 3 hours which was retained after 7 days was $12.9 \pm 2.0$ for the controls and $21.0 \pm 3.2$ for the EGF-treated mice thereby clearly indicating a markedly increased retention for the treated mice.

**Claims**

1. A composition suitable for use in vivo to enhance the level of iron in the bloodstream comprising epidermal growth factor and an iron-providing material.

2. A composition according to Claim 1, which is a pharmaceutical composition comprising epidermal growth factor and an iron-providing material together with a physiologically acceptable diluent or carrier.

3. A composition according to Claim 1 or 2, which is of injectable form.

4. A composition according to Claim 1 or 2, which is adapted for oral administration.

5. A composition according to Claim 1, which is a foodstuff comprising epidermal growth factor and an iron-providing material together with nutritional material.

6. A composition according to any of Claims 1 to 5, in which the epidermal growth factor is human, pig or mouse EGF.

7. A product comprising epidermal growth factor and an iron-providing material for simultaneous, separate or sequential use in effecting an increase in the level of iron in a patient's bloodstream.

8. A product according to Claim 7, in which the epidermal growth factor is human, pig or mouse EGF.

9. The use of epidermal growth factor for the manufacture of a medicament for use in effecting an increase in the level of iron in a patient's bloodstream.

10. The use according to Claim 9, in which the medicament is for administration to a patient which is a human, pig, bovine, goat, sheep or a horse.

11. The use according to Claim 9, in which the patient is a neonatal pig.

12. The use according to Claim 9, in which the patient is a human.

13. The use according to any of Claims 9 to 12, in which the epidermal growth factor is mouse EGF.

14. The use according to Claim 11, in which the epidermal growth factor is pig EGF.

15. The use according to Claim 12, in which the epidermal growth factor is human EGF.

16. A product comprising an iron-providing material and epidermal growth factor for use in therapy.

17. A product according to Claim 16, in which the epidermal growth factor is mouse EGF.

18. A product according to Claim 16, in which the epidermal growth factor is pig EGF.

19. A product according to Claim 16, in which the epidermal growth factor is human EGF.

**Patentansprüche**

1. Mittel, das für die in-vivo-Verwendung geeignet ist, um den Gehalt an Eisen in dem Blutstrom zu erhöhen, dadurch **gekennzeichnet**, daß es den epidermalen Wachstumsfaktor und ein Eisen lieferndes Material enthält.

2. Mittel nach Anspruch 1, dadurch **gekennzeichnet**, daß es ein pharmazeutisches Mittel ist, das den epidermalen Wachstumsfaktor und ein Eisen lieferndes Material zusammen mit einem physiologisch annehmbaren Verdünnungsmittel oder Träger enthält.

3. Mittel nach Anspruch 1 oder 2, dadurch **gekennzeichnet**, daß es in injizierbarer Form vorliegt.

4. Mittel nach Anspruch 1 oder 2, dadurch **gekennzeichnet**, daß es in einer für die orale Verabreichung geeigneten Form vorliegt.

5. Mittel nach Anspruch 1, dadurch **gekennzeichnet**, daß es ein Nahrungsmittel ist, welches den epidermalen Wachstumsfaktor und ein Eisen lieferndes Material zusammen mit einem Material, das einen Nährwert aufweist, enthält.

6. Mittel nach einem der Ansprüche 1 bis 5, dadurch **gekennzeichnet**, daß der epidermale Wachstumsfaktor humaner Schweine- oder Mäuse-EGF ist.

7. Produkt, dadurch **gekennzeichnet**, daß es einen epidermalen Wachstumsfaktor und ein Eisen lieferndes Material für die gleichzeitige, getrennte oder aufeinanderfolgende Verwendung enthält, um den Eisengehalt in dem Blutstrom eines Patienten zu erhöhen.

8. Produkt nach Anspruch 7, dadurch **gekennzeichnet**, daß der epidermale Wachstumsfaktor humaner Schweine- oder Mäuse-EGF ist.

9. Verwendung des epidermalen Wachstumsfaktors für die Herstellung eines Arzneimittels für die Verwendung zur Erhöhung des Eisengehalts in dem Blutstrom eines Patienten.

10. Verwendung nach Anspruch 9, wobei das Arneimittel für die Verabreichung an einen Patienten bestimmt ist, der ein Mensch, ein Schwein, ein Rind, eine Ziege, ein Schaf oder ein Pferd ist.

11. Verwendung nach Anspruch 9, wobei der Patient ein neonatales Schwein ist.

12. Verwendung nach Anspruch 9, wobei der Patient ein Mensch ist.

13. Verwendung nach einem der Ansprüche 9 bis 12, wobei der epidermale Wachstumsfaktor Mäuse-EGF ist.

**14.** Verwendung nach Anspruch 11, wobei der epidermale Wachstumsfaktor Schweine-EGF ist.

**15.** Verwendung nach Anspruch 12, wobei der epidermale Wachstumsfaktor humanes EGF ist.

**16.** Produkt, dadurch **gekennzeichnet**, daß es ein Eisen lieferndes Material und einen epidermalen Wachstumsfaktor enthält, für die Verwendung in der Therapie.

**17.** Produkt nach Anspruch 16, wobei der epidermale Wachstumsfaktor Mäuse-EGF ist.

**18.** Produkt nach Anspruch 16, wobei der epidermale Wachstumsfaktor Schweine-EGF ist.

**19.** Produkt nach Anspruch 16, wobei der epidermale Wachstumsfaktor humanes EGF ist.

**Revendications**

1.- Composition appropriée pour être utilisée in vivo afin d'augmenter le taux de fer dans le sang, contenant un facteur de croissance épidermique et une substance procurant du fer.

2.- Composition selon la revendication 1, qui est une composition pharmaceutique contenant un facteur de croissance épidermique et une substance procurant du fer avec un diluant ou un véhicule physiologiquement acceptable.

3.- Composition selon la revendication 1 ou la revendication 2, qui est une forme injectable.

4.- Composition selon la revendication 1 ou la revendication 2, qui est adaptée pour administration par voie orale.

5.- Composition selon la revendication 1, qui est un produit alimentaire contenant un facteur de croissance épidermique et une substance procurant du fer avec une substance nutritive.

6.- Composition selon l'une des revendications 1 à 5, dans laquelle le facteur de croissance épidermique est le facteur de croissance épidermique de l'homme, du porc ou de la souris.

7.- Produit contenant un facteur de croissance épidermique et une substance procurant du fer pour utilisation simultanée, séparée ou séquentielle, afin d'augmenter le taux de fer dans le sang du patient.

8.- Produit selon la revendication 7, dans lequel le facteur de croissance épidermique est le facteur de croissance épidermique de l'homme, du porc ou de la souris.

9.- Utilisation d'un facteur de croissance épidermique pour la fabrication d'un médicament destiné à être utilisé pour augmenter le taux de fer dans le sang d'un patient.

10.- Utilisation selon la revendication 9, dans laquelle le médicament est prévu pour être administré à un patient qui est un homme, un porc, un bovin, une chèvre, un mouton ou un cheval.

11.- Utilisation selon la revendication 9, dans laquelle le patient est un porc nouveau-né.

12.- Utilisation selon la revendication 9, dans laquelle le patient est une personne humaine.

13.- Utilisation selon l'une des revendications 9 à 12, dans laquelle le facteur de croissance épidermique est le facteur de croissance épidermique de la souris.

14.- Utilisation selon la revendication 11, dans laquelle le facteur de croissance épidermique est le facteur de croissance épidermique du porc.

15.- Utilisation selon la revendication 12, dans laquelle le facteur de croissance épidermique est le facteur de croissance épidermique de l'homme.

16.- Produit contenant une substance procurant du fer et un facteur de croissance épidermique pour être utilisé dans une thérapie.

17.- Produit selon la revendication 16, dans laquelle le facteur de croissance épidermique est le facteur de croissance épidermique de la souris.

18.- Produit selon la revendication 16, dans laquelle le facteur de croissance épidermique est le facteur de croissance épidermique du porc.

19.- Produit selon la revendication 16, dans laquelle le facteur de croissance épidermique est le facteur de croissance épidermique de l'homme.